# EUROPÄISCHE PATENTANMELDUNG

(11) **EP 2 353 507 A1**
(43) Veröffentlichungstag der Anmeldung: **10.08.2011**
(21) Anmeldenummer: 11000079.1
(22) Anmeldetag: 07.01.2011
(51) Int. Cl.: A61B 6/00

(54) **Schwenkvorrichtung für einen schwenkbaren C-Bogen eines Röntgengeräts**

(30) Priorität: 27.01.2010 DE 102010005787
(71) Anmelder: Richard Wolf GmbH, 75438 Knittlingen (DE)
(72) Erfinder: Müller, Klaus, 75438 Knittlingen (DE); Weisert, Willi, 75038 Oberderdingen (DE)
(74) Vertreter: Vollmann, Heiko

(57) **Zusammenfassung**

Eine Schwenkvorrichtung ist zum Verschwenken eines schwenkbaren C-Bogen (8) eines Röntgengeräts vorgesehen. Die Schwenkvorrichtung weist eine motorische Antriebseinheit (24) und einen an dem C-Bogen (8) konzentrisch zu dessen Schwenkachse (A) lösbar befestigbaren Adapter auf. Über den Adapter ist der C-Bogen (8) mit der Antriebseinheit (24) antriebsverbindbar. Darüber hinaus weist die Schwenkvorrichtung einen Winkellagegeber zur Erfassung des Schwenkwinkels des C-Bogen (8) auf.

## Beschreibung

Die Erfindung betrifft eine Schwenkvorrichtung für einen schwenkbaren C-Bogen eines Röntgengeräts.

Diagnostische Röntgengeräte der oben genannten Art werden unter anderem in Verbindung mit Therapievorrichtungen verwendet, um vor und ggf. während der Therapie den Therapieort im Körperinneren genau zu lokalisieren. Beispielsweise werden sie bei der extrakorporalen Therapie von Konkrementen, wie Nieren- oder Harnsteinen eingesetzt. Hierbei wird der Fokus einer zum Zertrümmern des Konkrements verwendeten Ultraschallquelle mit dem zuvor mittels des Röntgengeräts ermittelten Therapiepunkt zur Deckung gebracht. Zur Ermittlung des Therapiepunktes wird der zu behandelnde Patient zunächst mit dem Röntgengerät in zwei unterschiedlichen Ebenen durchleuchtet. Hierzu ist ein C-Bogen des Röntgengeräts, an dem aneinander direkt gegenüberliegend eine Röntgenstrahlenquelle und ein Bildverstärker angeordnet sind, typischerweise schwenkbar ausgebildet. So kann der Patient zum Beispiel zunächst in einer senkrechten Ebene und anschließend durch manuelles Schwenken des C-Bogens in einer zur der senkrechten Ebene geneigten Ebene durchleuchtet werden, woraus sich der Therapiepunkt im Schnittpunkt der Strahlengänge in diesen beiden Ebenen ergibt.

Das exakte Justieren des C-Bogens bei der Ermittlung des Therapiepunktes ist vergleichsweise aufwendig und verlangt eine gewisse Geschicklichkeit. Dies ist insbesondere dann der Fall, wenn der C-Bogen, dessen Position nach der Fokussierung der Ultraschallquelle auf den Therapieort eigentlich beizubehalten ist, versehentlich oder aus Therapiegründen vorsätzlich bewegt wird und dementsprechend wieder in seine Ausgangposition zurückgeschwenkt werden muss.

Vor diesem Hintergrund liegt der Erfindung die Aufgabe zu Grunde, eine Vorrichtung zu schaffen, mit der eine genauere Justierung des C-Bogens möglich wird und mit der die Justierung eines schwenkbaren C-Bogens eines Röntgengeräts erleichtert wird.

Gelöst wird diese Aufgabe durch eine Schwenkvorrichtung mit den in Anspruch 1 angegebenen Merkmalen. Vorteilhafte Weiterbildungen dieser Schwenkvorrichtung ergeben sich aus den Unteransprüchen, der nachfolgenden Beschreibung sowie der Zeichnung. Hierbei können gemäß der Erfindung, die in den Unteransprüchen angegebenen Merkmale jeweils für sich aber auch in Kombination die erfindungsgemäße Lösung gemäß Anspruch 1 weiter ausgestalten.

Die erfindungsgemäße Schwenkvorrichtung für einen bevorzugt lateral schwenkbaren C-Bogen eines Röntgengeräts weist eine motorische Antriebseinheit und einen an dem C-Bogen konzentrisch zu dessen Schwenkachse befestigten Adapter auf. Über den Adapter ist der C-Bogen mit der Antriebseinheit antriebsverbindbar, d. h., die Antriebseinheit kann mittels des Adapters so mit dem C-Bogen verbunden werden, dass die Bewegung eines bevorzugt als Drehantrieb ausgebildeten motorischen Antriebs der Antriebseinheit auf den C-Bogen übertragen wird. Zweckmäßigerweise ist die Antriebseinheit hierbei an einem solchen Teil des Röntgengeräts befestigt, der anders als der C-Bogen feststehend ausgebildet ist, so dass der C-Bogen relativ zu diesem Teil schwenkbar ist. Bei dem Antrieb kann es sich um einen Direktantrieb handeln, bei dem die Drehbewegung der Antriebswelle eines Antriebsmotors direkt auf den C-Bogen übertragen wird. Bevorzugt wird allerdings das zum Schwenken des C-Bogens erforderliche Drehmoment über ein dem C-Bogen vorgelagertes Getriebe auf den C-Bogen übertragen, wobei ein die Drehzahl des Antriebsmotors untersetzendes Getriebe eine besonders exakte Winkelpositionierung des C-Bogens ermöglicht.

Zur Erfassung des genauen Schwenkwinkels des C-Bogens, der bevorzugt in einem Winkelbereich von mindestens 60° und vorzugsweise ausgehend von einer senkrechten Normalstellung des C-Bogens in zwei einander entgegen gesetzten Richtungen quer zur Normalstellung in einem Winkelbereich von mindestens 30° liegen kann, ist bei der erfindungsgemäßen Schwenkvorrichtung ein Winkellagegeber bzw. ein Drehwinkelaufnehmer vorgesehen. Dieser Winkellagegeber ist zumindest mit einer Einrichtung zur Winkelanzeige signalverbunden. Daneben kann auch eine Signalverbindung des Winkellagegebers mit einer Steuervorrichtung vorgesehen sein, wobei die Steuervorrichtung vorteilhafterweise auch die Winkelanzeige beinhalten kann und zur Ansteuerung der Antriebseinheit dienen kann. Mit dem Winkellagegeber kann während des Schwenkens des C-Bogens die Winkellage des C-Bogens vorzugsweise kontinuierlich erfasst werden, so dass einer Bedienperson eines mit der erfindungsgemäßen Schwenkvorrichtung ausgestatteten Röntgengeräts jederzeit die aktuelle Winkelposition des C-Bogens bekannt ist. Die ist insbesondere dann von Vorteil, wenn der bereits auf den Einsatzort ausgerichtete C-Bogen versehentlich bewegt wird und in diesem Fall aufgrund der Kenntnis der genauen Winkelposition bei Ausrichtung auf den Einsatzort in einfacher Weise wieder entsprechend neu auf diesen Einsatzort ausgerichtet werden kann. In diesem Zusammenhang kann gegebenenfalls auch eine mit dem Winkellagegeber verbundene Steuervorrichtung eine entsprechende Ansteuerung der Antriebseinheit veranlassen, so dass der C-Bogen von der Antriebseinheit quasi automatisch wieder zurück in die geforderte Position bewegt werden kann.

Bei dem verwendeten Winkellagegeber kann es sich um einen solchen Sensor handeln, mit dem die Winkelstellung des C-Bogens beispielsweise berührungslos direkt ermittelt werden kann. Bevorzugt ist allerdings die Verwendung eines Winkellagegebers vorgesehen, der zur Erfassung des Drehwinkels eines mit dem Adapter verbindbaren Teils der Antriebseinheit angeordnet ist. Die ist insofern vorteilhaft, als der Winkellagegeber geschützt in einem Gehäuse der Antriebseinheit angeordnet werden kann.

Das mit dem Adapter verbindbare Teil der Antriebseinheit ist bevorzugt direkt mit dem an dem C-Bogen befestigten Adapter verbunden, so dass der Drehwinkel dieses Teils mit dem Drehwinkel des C-Bogens übereinstimmt. Die Art des Winkellagegebers ist prinzipiell beliebig, so lange sich mit ihm Drehwinkelpositionen oder zumindest Drehwinkeländerungen des mit dem Adapter verbundenen Teils ermitteln lassen. So kann der verwendete Winkellagegeber ein die Winkelposition bzw. Winkeländerung digital, analog, relativ oder absolut erfassender Sensor sein. Bevorzugt wird als Drehwinkelgeber ein Potentiometer verwendet, dessen Schleifer bzw. Gleitkontakt mit dem mit dem Adapter verbindbaren Teil der Antriebseinheit bewegungsgekoppelt ist. Zur Bewegungskopplung des Schleifers mit dem mit dem Adapter verbindbaren Teil der Antriebseinheit ist vorzugsweise ein Riementrieb vorgesehen. In besonders geeigneter Weise kann es sich bei dem Riementrieb um einen Zahnriementrieb handeln. Wahlweise kann es auch vorteilhaft sein, den Schleifer mittels einer Zahnradverbindung mit dem mit dem Adapter verbindbaren Teil der Antriebseinheit bewegungszukoppeln.

In weiterer vorteilhafter Ausgestaltung kann bei der erfindungsgemäßen Schwenkvorrichtung die Antriebswelle des motorischen Antriebs der Antriebseinheit, bei der es sich bevorzugt um die Antriebswelle eines Elektromotors handelt, über ein Schneckengetriebe mit dem mit dem Adapter verbindbaren Teil der Antriebseinheit verbindungsgekoppelt sein. Die Verwendung des Schneckengetriebes ermöglicht es, die Drehachse der Antriebswelle des Motors und die Drehachse des mit dem Adapter verbindbaren Teils normal zu einander auszurichten, wodurch sich eine vergleichsweise kompakte Bauform der Antriebseinheit realisieren lässt. Darüber hinaus lassen sich mit dem Schneckengetriebe verhältnismäßig große Drehzahluntersetzungen verwirklichen, wodurch sich mit der erfindungsgemäßen Schwenkvorrichtung eine besonders gute Positioniergenauigkeit des C-Bogens erzielen lässt.

Um eine sichere Drehmomentübertragung von der Antriebseinheit auf den C-Bogen zu ermöglichen, kann das mit dem Adapter verbindbare Teil der Antriebseinheit vorteilhaft Formschlusselemente aufweisen, die zum Eingriff in korrespondierende Formschlusselemente des Adapters vorgesehen sind. Typischerweise können umgekehrt auch an dem Adapter Formschlusselemente ausgebildet sein, die mit den entsprechenden Formschlusselementen der Antriebseinheit in Eingriff gebracht werden können. Die antriebseinheitsseitig und adapterseitig ausgebildeten Formschlusselemente sind typischerweise derart ausgebildet, dass sie zumindest eine formschlüssige Verbindung des Adapters mit dem damit verbindbaren Teil der Antriebseinheit quer zur Drehrichtung letztgenannten Teils schaffen.

In Weiterbildung der erfindungsgemäßen Schwenkvorrichtung kann diese vorteilhafterweise auch so ausgestaltet sein, dass sie ein schnelles, manuelles Verschwenken des C-Bogens ermöglicht, um so den Zugang zu dem Therapieort zu verbessern. Dementsprechend kann zweckmäßigerweise eine Rutschkupplung vorgesehen sein, die zwischen dem Antriebsmotor der Antriebseinheit und dem Adapter angeordnet ist. Diese Rutschkupplung ermöglicht es, den Antriebsstrang der Antriebseinheit zwischen dem Adapter und dem Antriebsmotor durch manuelles Verschwenken des C-Bogens kurzfristig zu trennen. Bevorzugt bildet hierzu das mit dem Adapter verbindbare Teil einen Teil einer Rutschkupplung.

Um ein Röntgengerät in Fällen, in denen der C-Bogen nicht so exakt ausgerichtet werden muss auch ohne Verwendung der erfindungsgemäßen Schwenkvorrichtung einsetzen zu können, ist die Antriebseinheit zweckmäßigerweise nach einem Lösen von dem Adapter in eine Stellung bewegbar, in der sie eine das Röntgengerät bedienende Person nicht behindert. Zu diesem Zweck kann die Antriebseinheit an einem an dem Röntgengerät befestigten Schwenkarm angeordnet sein und so bei Bedarf von dem C-Bogen weg geschwenkt werden. Bei Röntgengeräten, deren C-Bogen höhenverstellbar an einer an dem Röntgengerät vorgesehenen vertikal ausfahrbaren Trägersäule befestigt ist, kann der die Antriebseinheit tragende Schwenkarm zweckmäßigerweise an dieser Trägersäule oder einem mit der Trägersäule verfahrbaren Bauteil befestigt sein, um die Antriebseinheit unabhängig von der Höheneinstellung des C-Bogens mit dem daran befestigten Adapter verbinden zu können.

Bevorzugt ist der Adapter mittels einer Spannvorrichtung an dem C-Bogen befestigbar. Diese Spannvorrichtung ist vorzugsweise derart ausgestaltet, dass der Adapter an C-Bögen unterschiedlichen Querschnittsabmessungen befestigt werden kann. Dies ermöglicht es, die erfindungsgemäße Schwenkvorrichtung bei einer Vielzahl von Röntgengeräten unterschiedlicher Hersteller einzusetzen. Vorteilhaft kann als Spannvorrichtung ein einseitig offener Bügel mit dem daran angeordneten Adapter vorgesehen sein, der in einer den C-Bogen umgreifenden Stellung an diesem mittels geeigneter Spannmittel verspannt wird. Bei dieser Spannvorrichtung bestimmt lediglich die Weite des von dem Bügel umschlossenen Raums, an welchen C-Bögen die Spannvorrichtung befestigbar ist.

Nachfolgend ist die erfindungsgemäße Schwenkvorrichtung anhand eines in der Zeichnung dargestellten Ausführungsbeispiels näher erläutert. In der Zeichnung zeigt:
- Fig. 1: in schematischer Darstellung perspektivisch ein Röntgengerät mit einem C-Bogen und einer Schwenkvorrichtung zum Schwenken des C-Bogens,
- Fig. 2: in vergrößerter perspektivischer Einzelansicht den C-Bogen und die daran befestigte Schwenkvorrichtung des Röntgengeräts nach Fig. 1,
- Fig. 3: die Darstellung nach Fig. 2 mit einer von dem Schwenkarm entfernten Antriebseinheit der Schwenkvorrichtung und
- Fig. 4: schematisch und stark vergrößert eine Antriebseinheit der in den Figuren 1 bis 3 dargestellten Schwenkvorrichtung bei geöffnetem Gehäuse.

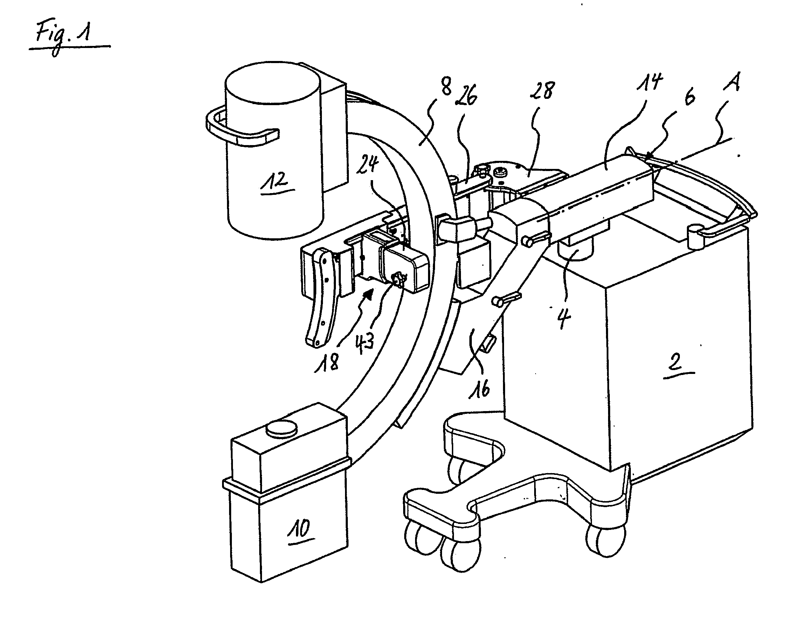

Bei dem in Fig. 1 dargestellten Röntgengerät handelt es sich um ein an sich bekanntes Gerät mit einem verfahrbaren Basiswagen 2, an dessen Oberseite eine in vertikaler Richtung verfahrbare Trägersäule 4 herausragt. An der Trägersäule 4 ist über einen abgewinkelten Arm 6 ein C-Bogen 8 des Röntgengeräts höhenverstellbar angeordnet. An dem C-Bogen 8 ist in üblicher Weise an einem unteren Ende eine Röntgenstrahlenquelle 10 und an einem oberen Ende ein Bildverstärker 12 angeordnet.

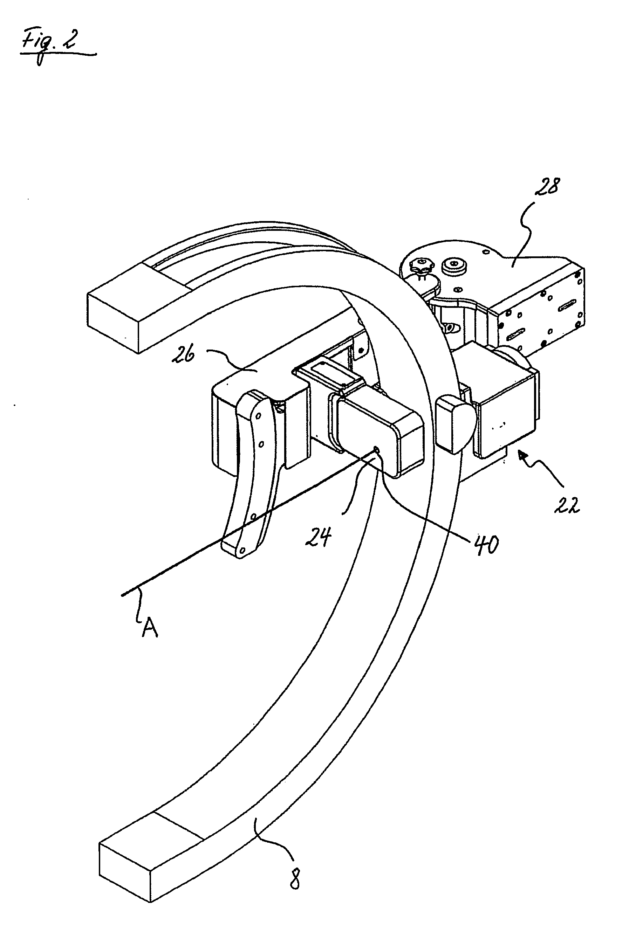

Der Arm 6, an dem der C-Bogen 8 befestigt ist, ist zweiteilig ausgebildet, und weist ein normal zur Längsausdehnung der Trägersäule 6 ausgerichtetes Armteil 14 und ein dazu abgeschrägt nach unten ausgerichtetes Armteil 16 auf. Das Armteil 16 ist an dem Armteil 14 um eine Längsachse A des Armteils 14 schwenkbar angelenkt. Somit ist auch der an dem Armteil 16 befestigte C-Bogen 8 um die Längsachse A des Armteils 14 schwenkbar. Die Längsachse A bildet demnach auch eine Schwenkachse A des C-Bogens 8. Zum Schwenken des C-Bogens 8 ist eine Schwenkvorrichtung 18 vorgesehen, deren Aufbau und Funktionsweise aus den Fig. 2 - 4 deutlich wird und nachfolgend anhand dieser Figuren näher erläutert wird.

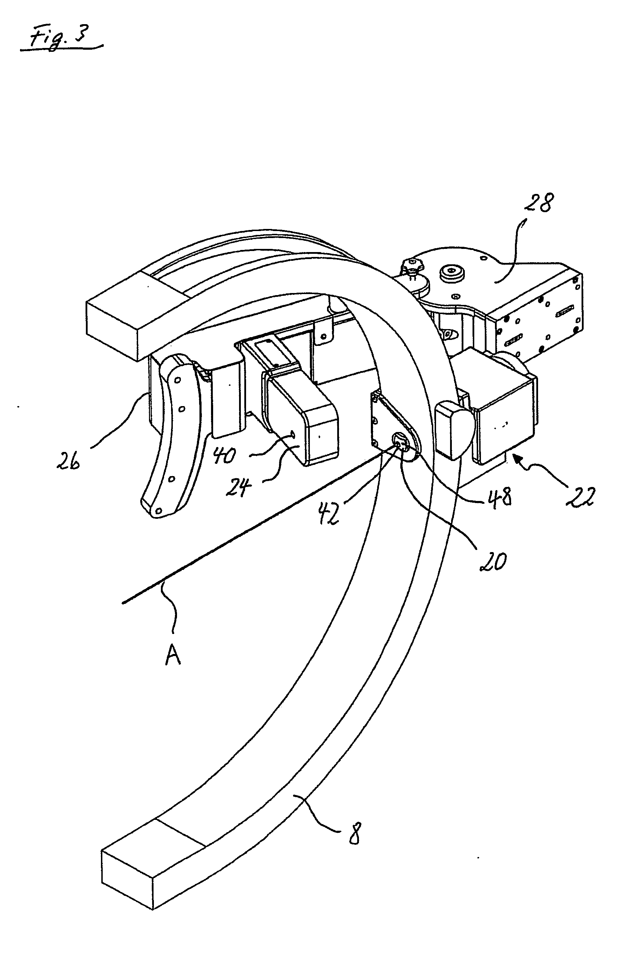

Die Schwenkvorrichtung 18 weist einen Adapter 20 auf (Fig. 3). Dieser Adapter 20 ist Teil eines bügelförmigen Klemmbauteils 22, das an dem C-Bogen 8 des Röntgengeräts befestigbar ist, wobei es den C-Bogen 8 derart umgreift, dass der Adapter 20 an der konkav gekrümmten und von dem Arm 6 abgewandten Seite des C-Bogens 8 konzentrisch zur Längsachse A, die auch die Schwenkachse des C-Bogens 8 bildet, angeordnet ist. Der Adapter 20 dient zur Bewegungskopplung des C-Bogens 8 mit einer Antriebseinheit 24 der Schwenkvorrichtung 18.

Die Antriebseinheit 24 ist an einem Schwenkarm 26 befestigt, der über ein Gelenk 28 an einer Längsseite des Armteils 14 des Röntgengeräts angelenkt ist. Insofern ist die Antriebseinheit 24 zusammen mit dem C-Bogen höhenverstellbar. Durch Schwenken des Schwenkarms 26 ist die Antriebseinheit 24 in eine Stellung bewegbar, in der der C-Bogen 8 über den Adapter 20 mit der Antriebseinheit 24 antriebsverbunden ist (Fig. 2), und in eine Stellung bewegbar, in der die Antriebsverbindung von C-Bogen 8 und Antriebseinheit 24 nicht besteht (Fig. 3).

Die Antriebseinheit 24 weist ein im Wesentlichen quaderförmiges Gehäuse 30 auf, das an dem Schwenkarm 26 derart befestigt ist, dass sich das Gehäuse 30 quer zur Längsausdehnung des Schwenkarms 26 erstreckt. In dem Gehäuse 30 der Antriebseinheit 24 befindet sich ein Elektromotor 32. Der Elektromotor 32 ist so in dem Gehäuse 30 angeordnet, dass sich die Antriebswelle 34 des Elektromotors 32 in Längsrichtung des Gehäuses 32 erstreckt.

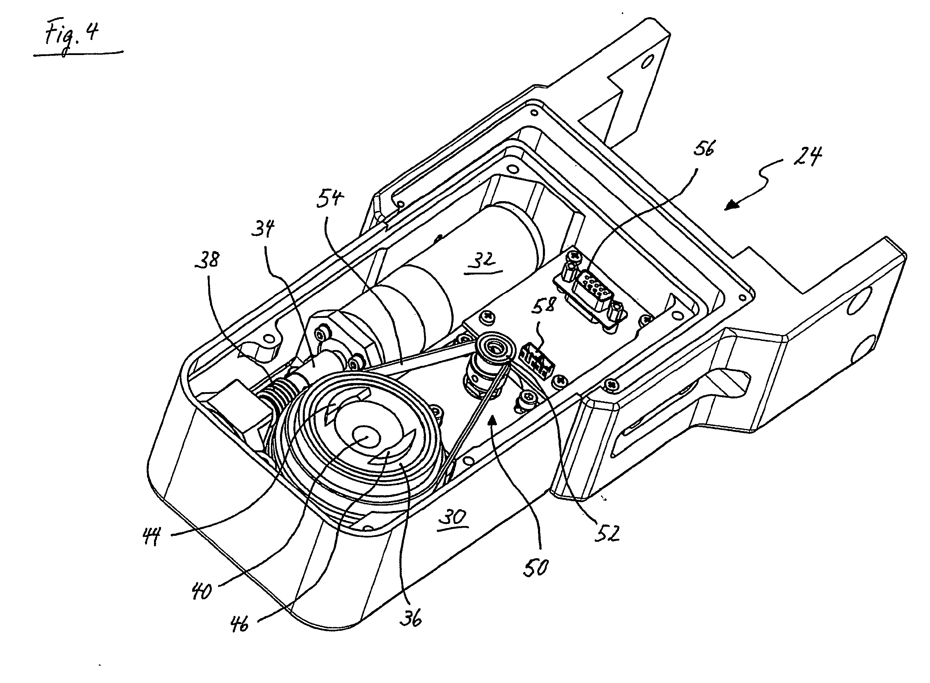

Der Elektromotor 32, der von einer nicht dargestellten Steuerung so ansteuerbar ist, dass seine Antriebswelle 34 in zwei entgegengesetzte Richtungen drehbar ist, dient zum Antrieb eines Antriebsrads 36, das mit dem Adapter 20 verbindbar ist und eine Drehbewegung der Antriebswelle 34 des Elektromotors 32 auf den C-Bogen 8 überträgt. In dem Gehäuse 30 ist das Antriebsrad 36 derart gelagert, dass seine Drehachse mit Abstand und normal zur Längsausdehnung der Antriebswelle 34 des Elektromotors 32 ausgerichtet ist. Zur Übertragung der Drehbewegung der Antriebswelle 34 auf das Antriebsrad 36 ist ein Schneckengetriebe vorgesehen. Dieses Schneckengetriebe weist eine an der Antriebswelle 34 des Elektromotors 32 angeordnete Schnecke 38 auf, in die ein in Fig. 4 unterhalb des Antriebsrads 36 mit dem Antriebsrad 36 drehgekoppeltes nicht sichtbares Schneckenrad kämmt.

Zur Befestigung der Antriebseinheit 24 an dem Adapter 20 weist das Antriebsrad 36 eine zentrale Bohrung 40 auf, die sich auch durch die Wandung des Gehäuses 30 erstreckt und an der von dem C-Bogen 8 abgewandten Außenseite des Gehäuses 30 mündet. Korrespondierend zur Bohrung 40 weist auch der Adapter 20 eine konzentrisch zur Längsachse A ausgerichtete Bohrung 42 auf. Die Bohrungen 40 und 42 dienen zur Aufnahme einer Schraube 43, die die Antriebseinheit 24 mit dem Adapter 20 verbindet. Um die Verbindung von Antriebseinheit 24 und Adapter 20 schnell manuell herstellen oder lösen zu können, weist die Schraube 43 bevorzugt einen Sternhandgriff auf.

Um eine sichere Drehmomentübertragung von dem Antriebsrad 36 auf den Adapter 20 zu ermöglichen, bildet das Antriebsrad 36 bei der Befestigung der Antriebseinheit 24 an dem Adapter 20 einen Formschluss mit dem Adapter 20. Hierzu weist das Antriebsrad 36 stirnseitig zwei einander beabstandet gegenüberliegende ringsegmentartige Vorsprünge 44 und 46 auf, die bei Anordnung des Antriebsrads 36 an dem Adapter 20 einen an dem Adapter 20 ausgebildeten Vorsprung 48 umgreifen.

In Fig. 4 nicht erkennbar ist das Antriebsrad 36 Teil einer zwischen dem Schneckengetriebe und dem Adapter 20 angeordneten Rutschkupplung und kann sich so bei einem gewissen auf das Antriebsrad 36 wirkenden Drehmoment von dem übrigen Antriebsstrang der Antriebseinheit 24 trennen. Dies ermöglicht es den ansonsten von der Antriebseinheit 24 schwenkbaren C-Bogen 8 ggf. auch manuell zu verschwenken.

Um die Schwenkposition des C-Bogens eindeutig bestimmen zu können, weist die Schwenkvorrichtung einen Winkellagegeber 50 auf. Dieser Winkellagegeber dient zur Erfassung der Drehstellung des Antriebsrads 36 und damit zur Erfassung des Schwenkwinkels des mit dem Antriebsrad 36 bewegungsgekoppelten C-Bogens 8. Bei dem Winkellagegeber handelt es sich um ein Potentiometer 50, das in dem Gehäuse 30 der Antriebseinheit 24 angeordnet ist. Das Potentiometer 50 weist eine Welle 52 auf, die über einen Riemen 54 mit dem Antriebsrad 36 bewegungsgekoppelt ist. Alternativ zu der in Fig. 4 dargestellten Ausgestaltung kann die Welle 52 auch über eine Zahnradverbindung mit dem Antriebsrad 36 bewegungsgekoppelt sein.

### Bezugszeichenliste

- 2 -: Basiswagen
- 4 -: Trägersäule
- 6 -: Arm
- 8 -: C-Bogen
- 10 -: Röntgenstrahlenquelle
- 12 -: Bildverstärker
- 14 -: Armteil
- 16 -: Armteil
- 18 -: Schwenkvorrichtung
- 20 -: Adapter
- 22 -: Klemmbauteil
- 24 -: Antriebseinheit
- 26 -: Schwenkarm
- 28 -: Gelenk
- 30 -: Gehäuse
- 32 -: Elektromotor
- 34 -: Antriebswelle
- 36 -: Antriebsrad
- 38 -: Schnecke
- 40 -: Bohrung
- 42 -: Bohrung
- 43 -: Schraube
- 44 -: Vorsprung
- 46 -: Vorsprung
- 48 -: Vorsprung
- 50 -: Winkellagegeber, Potentiometer
- 52 -: Welle
- 54 -: Riemen
- 56 -: Stecker
- 58 -: Stecker

- A -: Längsachse, Schwenkachse

## Patentansprüche

1. Schwenkvorrichtung für einen schwenkbaren C-Bogen (8) eines Röntgengeräts, mit einer motorischen Antriebseinheit (24) und mit einem an dem C-Bogen (8) konzentrisch zu dessen Schwenkachse (A) lösbar befestigbaren Adapter (20), über den der C-Bogen (8) mit der Antriebseinheit (24) antriebsverbindbar ist, sowie mit einem Winkellagegeber (50) zur Erfassung des Schwenkwinkels des C-Bogens (8).

2. Schwenkvorrichtung nach Anspruch 1, bei der der Winkellagegeber (50) zur Erfassung des Drehwinkels eines mit dem Adapter (20) verbindbaren Teils der Antriebseinheit (24) angeordnet ist.

3. Schwenkvorrichtung nach Anspruch 2, bei der der Winkellagegeber (50) ein Potentiometer (50) ist, dessen Schleifer mit dem mit dem Adapter (20) verbindbaren Teil der Antriebseinheit (24) vorzugsweise mittels eines Riementriebs und weiter bevorzugt mittels eines Zahnriementriebs bewegungsgekoppelt ist.

4. Schwenkvorrichtung nach Anspruch 3, bei der der Schleifer des Potentiometers (50) mit dem mit dem Adapter (20) verbindbaren Teil der Antriebseinheit (24) vorzugsweise mittels einer Zahnradverbindung bewegungsgekoppelt ist.

5. Schwenkvorrichtung nach einem der vorangehenden Ansprüche, bei der die Antriebswelle (34) eines Elektromotors (32) über ein Schneckengetriebe mit dem mit dem Adapter (20) verbindbaren Teil der Antriebseinheit (24) bewegungsgekoppelt ist.

6. Schwenkvorrichtung nach einem der vorangehenden Ansprüche, bei der das mit dem Adapter (20) verbindbaren Teil der Antriebseinheit (24) Formschlusselemente aufweist, die zum Eingriff in korrespondierende Formschlusselemente des Adapters (20) vorgesehen sind.

7. Schwenkvorrichtung nach einem der vorangehenden Ansprüche, bei der zwischen einem Antriebsmotor der Antriebseinheit (24) und dem Adapter (20) eine Rutschkupplung angeordnet ist.

8. Schwenkvorrichtung nach einem der vorangehenden Ansprüche, bei der das mit dem Adapter (20) verbindbare Teil der Antriebseinheit (24) einen Teil einer Rutschkupplung bildet.

9. Schwenkvorrichtung nach einem der vorangehenden Ansprüche, bei der die Antriebseinheit (24) an einem an dem Röntgengerät befestigten Schwenkarm ((26) angeordnet ist.

10. Schwenkvorrichtung nach einem der vorangehenden Ansprüche, bei der der Adapter (20) mittels einer Spannvorrichtung an dem C-Bogen (8) befestigbar ist.

11. Schwenkvorrichtung nach Anspruch 10, bei der der Adapter an einem einseitig offenen Bügel angeordnet ist, der an dem C-Bogen eines Röntgengeräts verspannbar ist.
